## Europäisches Patentamt
## European Patent Office
## Office européen des brevets

(19)

(11) Publication number: **0 066 377**
**A1**

(12)

# EUROPEAN PATENT APPLICATION

(21) Application number: **82302331.2**

(51) Int. Cl.³: **C 12 P 19/06**

(22) Date of filing: **07.05.82**

(30) Priority: **22.05.81 GB 8115856**

(43) Date of publication of application:
**08.12.82 Bulletin 82/49**

(84) Designated Contracting States:
**AT BE CH DE FR GB IT LI LU NL SE**

(71) Applicant: **Kelco Biospecialties Limited**
**22 Henrietta Street**
**London WC2E 8NB(GB)**

(72) Inventor: **Jarman, Trevor Rodney**
**18, Moss Drive**
**Haslingfield Cambridgeshire(GB)**

(74) Representative: **Ablewhite, Alan James et al,**
**MARKS & CLERK 57/60 Lincoln's Inn Fields**
**London WC2A 3LS(GB)**

(54) **Process for xanthan gum production.**

(57) Xanthan gum is produced by an improved process of fermenting an assimilable carbon source by an organism of the genus *Xanthomonas* comprising limiting the trace elements Fe, Mn, Zn, Cu, Co and B as follows:

| | | |
|----|--------|------|
| Fe | 0.1 | - 10 |
| Mn | 0.01 | - 1 |
| Zn | 0.01 | - 1 |
| Cu | 0.001 | - 1 |
| Co | 0.001 | - 1 |
| B | 0.0005 | - 1 |

with the proviso that the ratio Re: Mn ranges from 40: 1 to 2: 1.

"PROCESS FOR XANTHAN GUM PRODUCTION"

The present invention relates to the production of xanthan gum.

Xanthan gum is a polysaccharide elaborated by many species of the genus Xanthamonas. The gum has interesting rheological properties and is used particularly as a thickener in oil drilling and in foodstuffs.

Industrial production of xanthan gum is generally by a batch process, in which a culture of a polysaccharide-producing strain of Xanthamonas is inoculated into a nutrient medium in a fermentation vessel and allowed to ferment for up to several days. Thereafter the gum is usually isolated from the fermented broth by addition of an organic solvent to precipitate the gum.

An alternative to batch production is continuous production, in which after inoculation and initial growth of the bacterium, fresh nutrient medium is continuously added to the fermentation vessel and a corresponding amount of fermented broth is continuously withdrawn. The advantages of continuous xanthan gum production, especially regular production, ease of control and smaller fermentation vessel, have been recognized for many years. However, for various reasons, particularly practical difficulties in maintaining extended, productive fermentation, batch production still predominates.

Much of the research on xanthan gum production has been carried out in the USA, particularly at the Northern Regional Research Laboratory of the US Department of Agriculture at Peoria, Illinois, USA. Proceeding from the pioneering work on the strain NRRL B-1459 of Xanthomonas campestris, R W Silman and P Rogovin of the Peoria Laboratory demonstrated that continuous fermentation was feasible (Biotechnology and Bioengineering, 12, 75 (1970). In a later article (ibid, 14, 23 (1972)) they reported further study on the possibilities for continuous fermentation, with a maximum xanthan production rate of 0.84 g/h/kg. This figure is relatively low, and moreover the resultant fermentation broth contained only 0.6% xanthan and had a viscosity of only 800 cp (Table III on page 27). Higher xanthan contents and higher viscosity products are reported, but at lower xanthan production rates.

More generally, there are numerous continuous processes for xanthan production which are described in the patent and other literature. Examples are contained for instance in US Patents No. 3,328,262 and No. 3,485,719, as well as in UK Patent Specification No. 1,512,536 and 2,008,138. In practice, each of the known processes gives a product with different characteristics, reflecting variations in the nature of the xanthan polysaccharide.

We have been studying the production of xanthan gum by continuous procedures, with a view to obtaining with high productivity a product having good rheology and being acceptable for use in foods intended for human consumption.

According to the present invention, we provide a continuous process for the production of a polysaccharide, xanthan gum, by continuously culturing a polysaccharide-producing strain of bacterium of the genus Xanthamonas in a fermentation medium, wherein the fermentation medium contains certain trace elements at controlled, low levels. It is by the use of the controlled trace elements at low levels that we find it possible to improve the purity and other characteristics of a xanthan gum product obtained at relatively high productivity.

More specifically, in accordance with the invention, we use for the continuous cultivation a fermentation medium which contains low levels of iron, manganese, zinc, copper, cobalt and boron.

Considered generally, the level of the trace elements had not previously been identified as a possible factor for optimising xanthan gum production. Surprisingly, these elements appear to play a key role in continuous processes.

Silman and Rogovin make no mention of the trace elements in their papers to which reference has been made above. Similarly the trace elements are not considered in the US Patent No. 3,485,719. In the US Patent No. 3,328,262 it is simply indicated that "suitable trace elements will normally be used", without specifying their nature or suitable levels. The UK Patent No 1,512,536 says that the medium should contain trace elements, though the Examples do not give much detail on this point and simply say that the medium contained "traces of various metals such as zinc, copper, cobalt, iron, manganese and molybdenum". Finally, in the literature mentioned on continuous fermentation, the UK Patent Specification No 2003138 identifies in Example 1 a suitable trace element mixture.

In the art of batch production, there is UK Patent No. 1,531,970 which describes the use of controlled levels of manganese and iron, as well as of chelated calcium. The manganese level is set at about 0.75 to 60 ppm and the iron level at about 0.25 to 20 ppm. Nothing is said about the level of zinc. In practice, the manganese is employed in the examples at 9.75 ppm and the iron at about 2 ppm. The use of these levels in UK 1531970 is one of several factors in a batch process designed for producing a fermented broth which is suitable for direct use in oil recovery. The aim is a broth substantially free of insoluble matter having a particle size above 3 microns.

In contrast, the present invention is a continuous process wherein the level of manganese is less than that of iron.

For preference, the fermentation medium for a continuous process of the invention contains the following levels of the elements specified in Table 1 ('ppm' in this specification being parts by weight of the element per million parts by volume of the medium ).


## Table 1

| Trace Element | Preferred Level (ppm) | Most Preferred Level (ppm) |
|---|---|---|
| Fe | 0.1 to 10 | 0.5 to 5 |
| Mn | 0.01 to 1 | 0.1 to 1 |
| Zn | 0.01 to 1 | 0.1 to 0.5 |
| Cu | 0.001 to 1 | 0.01 to 0.1 |
| Co | 0.001 to 1 | 0.01 to 0.1 |
| B | 0.0005 to 1 | 0.005 to 0.05 |

More generally, the present invention employs iron at less than 10 ppm, manganese at less than 1 ppm, with the iron:manganese ppm ratio preferably being from 40:1 to 2:1.

In preparing a fermentation medium for use in the present process, the various trace elements are suitably added as water-soluble compounds, especially inorganic salts such as $FeCl_2.6H_2O$ or $FeSO_4.7H_2O$ for iron; $MnCl_2.6H_2O$ or $MnSO_4.4H_2O$ for manganese;

$ZnSO_4.7H_2O$ for zinc; $CuCl_2.2H_2O$ or $CuSO_4.5H_2O$ for copper; $CoCl_2.6H_2O$ or $CoSO_4.7H_2O$ for cobalt; and $H_3BO_3$ for boron.

In adding the trace elements, it is necessary to make due allowance for the amounts present in the other components. It is greatly preferred to employ demineralized water prepared for example by passing tap water through an ion exchanger. Equally, it is greatly preferred to analyse the medium after it has been made up, in order to confirm that the controlled, low levels of trace elements have not been exceeded.

Apart from the trace elements for which levels have already been specified, the fermentation medium will contain sources of assimilable carbon and of assimilable nitrogen, together with sources of other elements such as one or more of phosphorus magnesium, calcium, potassium and sulphur. The ingredients can be added using conventional sources at conventional levels.

The carbon source can be a sugar or other carbohydrate or a complex naturally-occurring source, with glucose being preferred, particularly in an amount of 30 to 70 g/l.

The nitrogen can be added as an ammonium salt or as a nitrate, but advantageously at least some of the nitrogen is supplied from a complex, naturally-occurring mixture, for instance yeast extract. Yeast extract, even at relatively low levels of 0.05 to 0.5 g/l, can usefully contribute growth factors, vitamins, amino acids and other, unidentified complex organic compounds.

The other elements such as phosphorus, sulphur and further metals can be added as water-soluble inorganic salts.

With a continuous process it necessarily follows that some requirement of the bacterium is a limiting factor. It is not essential to know the identity of the limiting factor, but on the other hand there are normally constraints on reformulating a fermentation medium if the limiting factor is unknown. For preference, the limiting factor is known and is nitrogen or sulphur.

The medium preferably has a pH of 6 to 8, and will usually be at about pH 7. Control of pH can be achieved by metered addition of acid or alkali as required to maintain the desired value.

In proceeding with a continuous process embodying the present invention, it is necessary first to have an inoculum of the polysaccharide-producing bacterium. Procedures for producing such inocula are well documented in the literature, and it is unnecessary to give details of the conventional procedures in this specification. For preference, the bacterium is of the species X. campestris, and furthermore the bacterium is preferably adapted to the fermentation medium as part of the growing up procedure. Thus, a culture of the productive strain is successively grown in stages using larger volumes of medium, with at least the final stage of inoculum production employing substantially the same medium as in the polysaccharide-producing strain.

After inoculation of fermentation medium and growing up to a satisfactory cell mass and product concentration, the present process is set in operation by adding fresh medium and drawing off fermented medium. The dilution rate is preferably 0.01 to 0.12 $h^{-1}$, more preferably 0.02 to 0.08 $h^{-1}$, say 0.04 to 0.06 $h^{-1}$. The fermentation temperature is suitably 25 to 35$^{o}$C, for instance 29 to 32$^{o}$C, but other temperatures can be used.

Oxygen has to be supplied during the fermentation because the Xanthomonas bacteria are strict aerobes. For preference the air flow and mixing conditions are preferably adjusted to keep the dissolved oxygen tension at above the levels which cause oxygen limitation and below the levels which cause oxygen toxicity.

The polysaccharide in the medium withdrawn from the fermentation vessel can be further processed, if desired, for example in order to give a solid product. For a food-grade material, it is preferred first to heat the fermented medium to say 100 to $130^{\circ}$C for 1 to 15 minutes, especially 120 to $125^{\circ}$ C for about 2 minutes, and thereafter to add an organic solvent such as isopropanol to precipitate the xanthan gum which may be filtered off or otherwise separated.

By adoption of the present process, it is possible to obtain with high productivity a xanthan gum of high viscosity and purity.

The present invention is illustrated by the following non-limiting examples. For the Examples, and the accompanying Comparative Examples, the following standard procedures were employed:

Product concentration determination (TPM)

An aliquot of culture (about 20g) was accurately weighed into a suitable glass vessel. Polymer, cells and some salts were precipitated with 2 volumes of isopropanol. The precipitate was separated by filtration after 10 minutes, through a pre-weighed glass fibre filter circle. The papers were dried to constant weight either in a vacuum oven at 45° C for 24 hours or under an infra-red lamp, then reweighed to give a total precipitate matter (TPM) determination. A larger aliquot was precipitated at steady state.

Polysaccharide Analyses

Samples of TPM or cell-free xanthan (CFX) were milled to ensure homogeneity and analysed for moisture content, sulphated ash, total mitrogen, pyruvate and acetyl.

Rheology Measurements

The rheology of culture broths were measured routinely throughout fermentation. Approximately 0.1 g of milled polymer was placed in a pre-weighed glass vial (including the cap). The polymer was dried under vacuum, over phosphorous pentoxide, overnight. The vial was capped under an atmosphere of dry nitrogen and reweighed.

A 1% solution was prepared from the dried polymer, in distilled water. Rheology (K and n values) was measured using a Wells-Brookfield 'cone and plate' viscometer (Model HBT) at 25° C.

$E_{540}$

The turbidity of diluted culture broths (1:40 by volume dilution) assessed as concentration.

Examples 1 to 5 and Comparative Examples 1 to 4

A continuous culture apparatus was designed to facilitate adequate mixing of a xanthan-containing broth. Two impellers with 6 curved blades (13 cm diameter) were fitted to a 5 litre top-stirred fermenter. The impellers were positioned so that the blades were backswept from the direction of rotation and 10 cm apart (bottom impeller at lowest point on the impeller shaft). As the impeller diameter was almost as great as the glass body of the fermenter, temperature was controlled using a 'serpent' of stainless steel tubing (6 mm o.d. and 4 mm i.d.) fitted against the inside of the glass body and connected to a Temperature Controller. The temperature-monitoring probe was introduced through the base plate. The temperature was maintained at about $30^O$C. pH was monitored using an electrode fitted in a side arm of the fermenter body and maintained at about 7.0 by automatic addition of 2M NaOH. Air was introduced through the centre of the base plate directly underneath the bottom impeller.

A weir was introduced through the top-plate and the length adjusted to give a working volume of about 2 litre.  For continuous operation, medium was continuously added and effluent continuously removed using  peristaltic pumps.

A shake flask containing 100 ml of a 24h culture of a laboratory stock of X. campestris ATCC 13951 in MYGP medium (malt extract, 3 $gl^{-1}$; yeast extract 3 $gl^{-1}$; glucose, 10 $gl^{-1}$; peptone, 5 $gl^{-1}$; agar, 20 $gl^{-1}$) was used to inoculate about 2 litre of stirred medium.  The composition of each medium is listed in Table 2.

Table 2

| Component | | Example | | | | | Comparative Example | | | | Component | |
|---|---|---|---|---|---|---|---|---|---|---|---|---|
| | | 1 | 2 | 3 | 4 | 5 | 1 | 2 | 3 | 4 | | |
| Acetic acid | $gl^{-1}$ | 0.45 | 0.45 | 0.45 | 0.45 | 0.45 | 0.45 | — | — | — | Acetic acid | $gl^{-1}$ |
| Citric acid | | 1.0 | 1.0 | 1.0 | 1.25 | 1.0 | 1.0 | 1.0 | 1.0 | 1.0 | Citric acid | |
| $Mg(OH)_2$ | | 0.0775 | 0.07 | 0.07 | 0.0848 | 0.0775 | 0.1 | 0.048 | 0.034 | 0.0775 | $Mg(OH)_2$ | |
| $Ca(OH)_2$ | | 0.05 | 0.05 | 0.05 | 0.05 | 0.05 | 0.05 | — | 0.025 | 0.05 | $Ca(OH)_2$ | |
| $MgSO_4.7H_2O$ | | 0.058 | 0.086 | 0.087 | 0.027 | 0.058 | — | — | 0.062 | 0.058 | $MgSO_4.7H_2O$ | |
| $K_2HPO_4$ | | 1.0 | 1.0 | 1.0 | 1.0 | 1.0 | 1.0 | 0.782 | 0.5 | 1.0 | $K_2HPO_4$ | |
| $(NH_4)_2HPO_4$ | | 2.62 | 2.38 | 2.38 | 2.36 | 3.0 | 3.75 | 3.02 | 3.77 | 3.77 | $(NH_4)_2HPO_4$ | |
| Yeast Extract | | — | — | 0.2 | — | — | — | — | 0.2 | — | Y.E. | |
| Glucose | | | | | | | | | | | Glucose | $gl^{-1}$ |
| Dexyme 081 | | 62 | 62 | 60 | 55 | 59 | 44 | 38.5 | 43 | 62 | Dexyme 081 | |
| Meritose | | — | — | — | — | — | — | — | — | — | Meritose | |
| | $mgl^{-1}$ | | | | | | | | | | | $mgl^{-1}$ |
| $MnSO_4.4H_2O$ | | 0.55 | 1.1 | 1.1 | 1.1 | 1.1 | 22 | — | — | 1.1 | $MnSO_4.4H_2O$ | |
| $ZnSO_4.7H_2O$ | | 0.71 | 1.42 | 0.71 | 1.42 | 1.42 | 28.4 | — | — | — | $ZnSO_4.7H_2O$ | |
| $CuSO_4.5H_2O$ | | 0.125 | 0.25 | 0.25 | 0.25 | 0.25 | 5.0 | — | — | 0.25 | $CuSO_4.5H_2O$ | |
| $CoSO_4.7H_2O$ | | 0.14 | 0.28 | 0.28 | 0.28 | 0.28 | 5.66 | — | — | 0.28 | $CuSO_4.7H_2O$ | |
| $H_3BO_4$ | | 0.03 | 0.06 | 0.06 | 0.06 | 0.06 | 1.2 | 0.553 | — | 0.06 | $H_3BO_4$ | |
| $FeSO_4.7H_2O$ | | 17.8 | 3.56 | 3.56 | 35.6 | 35.6 | 89 | 0.0143 | — | 35.6 | $FeSO_4.7H_2O$ | |

The glucose syrup Dexyme 081 was selected because of its purity, being low in trace elements. In ppm, the syrup contained the following: Zn, less than 0.01; Cu, less than 0.2; Mn, less than 0.04; Co, less than 0.08; Fe, less than 0.18.

The yeast extract was a special low salts yeast extract, and contained in ppm the following: Zn, 60; Cu, 10; Mn, 15; Co, less than 5, Fe, 100.

The other reagents were of analytical grade.

After batch growth of 24 h, the continuous addition of defined medium was commenced. The dilution rate was about $0.04 \; h^{-1}$.

A steady state sample was harvested only after at least 3 days of growth with constant measurements. The gum in the sample was precipitated by addition of excess isopropyl and assessed before ('NHT') or after ('HT') a heat treatment at 125° C for 2 minutes.

The fermentation conditions and product analyses are given in Table 3.

## Table 3

### EXAMPLE

| CONDITIONS | 1 | | 2 | | 3 | | 4 | | 5 | |
|---|---|---|---|---|---|---|---|---|---|---|
| Limitation (concentration) | $S=0.012gl^{-1}$ | | $S=0.012gl^{-1}$ | | $S=0.012gl^{-1}$ | | $S=0.009gl^{-1}$ | | $S=0.012gl^{-1}$ | |
| Product concentration $(gl^{-1})$ | 26.8 | | 32 | | 27.6 | | 22.7 | | 26.9 | |
| Cell concentration ($E_{540}$ units) | 4.6 | | 6.8 | | 5.34 | | — | | 4.8 | |
| Dry cell wt. $(gl^{-1})$ | 3 | | 3.8 | | 3.5 | | | | 3.6 | |
| Broth rheology K(n)cps | 35124 (.20) | | 39764 (.19) | | 28796 (.22) | | 41200 | | 37007 (.19) | |
| q $O_2$(m.moles $(g.h)^{-1}$) | 3.4 | | 3.8 | | 4.5 | | 7.3 | | 6.33 | |
| Polymer to cell ratio | 7.6 | | 7.4 | | 6.9 | | — | | 6.47 | |
| **PRODUCT** | NHT | HT | NHT | HT | NHT | HT | NHT | HT | NHT | HT |
| Rheology of product in salt, K(n)cps   1.0% | 11204 (.14) | 18336 (.01) | 10285 (.13) | 15481 (.08) | 10450 (.14) | 14386 (.08) | 7441 (.19) | 13554 (.10) | 13272 (.12) | 17321 (.07) |
| 0.3% | 621 (.31) | 1548 (.22) | 538 (.39) | 1320 (.24) | 691 (.35) | 1682 (.20) | 360 (.44) | 346 (.45) | 716 (.36) | 1569 (.22) |
| 0.1% | 46 (.60) | 192 (.39) | 38 (.63) | 110 (.47) | 63 (.55) | 179 (.39) | 30 (.65) | 30 (.65) | 77 (.52) | 142 (.45) |
| Decarboxylation (corrected)% | 4.2 | 4.2 | 4.1 | 4.4 | 4.1 | 4.4 | 4.02 | 4.19 | 4.3 | 4.41 |
| Ash (%) | | 11.8 | 9.4 | 8.6 | 12.45 | 12.7 | 14.56 | 14.38 | 10.3 | 11.09 |
| N (%) | 1.32 | 1.39 | 1.4 | 1.4 | — | 1.0 | 1.43 | 1.39 | 1.83 | 1.70 |
| Pyruvate (%) | 7.0 | 6.5 | 5.3 | 5.1 | 5.4 | 6.4 | 6.1 | 6.9 | 6.7 | 5.8 |
| Moisture (%) | 4.0 | 0.6 | 6.7 | 7.0 | 9.0 | 7.1 | 11.04 | 11.14 | 9.2 | 9.11 |

Table 3

| conditions | 1 | | 2 | | 3 | | 4 | |
|---|---|---|---|---|---|---|---|---|
| Limitation (concentration) | N(S=0.018gl⁻¹) | | S=0.012gl⁻¹ | | S=0.012gl⁻¹ | | S=0.012gl⁻¹ | |
| Product concentration (gl⁻¹) | 23.1 | | 19.7 | | 22.6 | | 22.0 | |
| Cell concentration ($E_{540}$ units) | 4.52 | | 3.88 | | 5.2 | | 3.0 | |
| Dry cell wt. (gl⁻¹) | | | 2.25 | | 3.4 | | 2.8 | |
| Broth rheology K(n)cps | 45076 (.15) | | 24139 (.14) | | 43133 (.13) | | 31817 (.18) | |
| $qO_2$(m.moles (g.h)⁻¹) | 3.57 | | 7.4 | | 6.53 | | 6.8 | |
| Polymer to cell ratio | 5.6 | | 7.76 | | 5.65 | | 6.9 | |
| PRODUCT | NHT | HT | NHT | HT | NHT | HT | NHT | HT |
| Rheology of product in salt, K(n)cps — 1.0% | 8567 (.18) | 18826 (.06) | 10400 (.16) | 12582 (.13) | 9691 (.17) | 17102 (.09) | 10573 (.15) | 13987 (.11) |
| 0.3% | 459 (.41) | 1304 (.25) | 333 (.48) | 575 (.37) | 1320 (.26) | 1384 (.24) | 688 (.38) | 1396 (.25) |
| 0.1% | 43 (.60) | 101 (.49) | 36 (.63) | 51 (.58) | 63 (.55) | 100 (.50) | 69 (.53) | 161 (.43) |
| Decarboxylation % [a] | 4.36 | 4.20 | 4.27 | 4.37 | 3.9 | 4.06 | 4.0 | 4.2 |
| Ash (%) [a] | 10.39 | 8.32 | 10.47 | 10.67 | 6.51 | 7.66 | 13.2 | 12.8 |
| N (%) [a] | 2.21 | 2.08 | 2.18 | 2.18 | 1.86 | 1.96 | 1.72 | 1.69 |
| Pyruvate (%) | 0.9 | 1.40 | 3.4 | 3.4 | 1.3 | 0.6 | 7.2 | 6.7 |
| Moisture (%) | 5.76 | 7.62 | 5.28 | 12.27 | 10.28 | 3.21 | 6.04 | 6.1 |

- 17 -

In Example 1, the fermentation produced an acceptable TPM and $E_{540}$, and the polymer:cell ratio was 7.6. The product had excellent rheological properties and an acceptable pyruvate content.

In Example 2, with double the level of manganese, zinc, copper, cobalt and boron, and with one-fifth the level of iron, the $E_{540}$, dry cell weight and TPM were excellent. The product had good rheological properties and the pyruvate content and decarboxylation assay were also high.

For Example 3, yeast extract was included in the medium. A steady state was obtained for this fermentation with a productivity of 1.21 $gl^{-1}$. The polymer:cell ratio was 6.9 and the product had a high pyruvate content and decarboxylation assay. In addition to good rheological properties, the heat-treated product had an inherent viscosity of 26.8 $dl.g^{-1}$ which compares very favourably with other samples and indicates a high molecular weight polymer.

For Example 4, less sulphate was included to test whether product purity, particularly polymer:cell ratio, N content and decarboxylation assay could be improved by lowering the cell mass when the sulphur concentration decreased. At 75% sulphur there was a corresponding lowering in $E_{540}$, indicating that the process was indeed sulphur-limited. The $QO_2$ was unchanged and the TPM decreased as expected. The product:cell ratio as based on TPM:$E_{540}$ ratio was unchanged compared to cultures with sulphur concentration of 0.012 $gl^{-1}$ run at a similar dissolved oxygen concentration. Furthermore, no great changes were observed in the nitrogen content or decarboxylation assay of the product. The decarboxylation assay was possibly depressed by glucose contamination of the product.

The results of Example 5 gave equally encouraging results. A TPM of about 27 $gl^{-1}$ was obtained, thus indicating good productivity. The $QO_2$ and polymer:cell ratio were high.

Comparison Example 1 typifies our research with the use of a higher concentration of trace elements. The small scale fermentations of this type were characterised by low TPM, productivity and conversion efficiency. In addition product rheology was poor unless heat-treated, which might reflect the low purity of the product since the products failed in nitrogen content and decarboxylation assay. The polymer: cell ratio and respiration rate were both low.

For Comparison Example 2, the culture was grown in the absence of any added trace elements or organic nutrient supplement and produced a low yield of xanthan. However, the $QO_2$ and polymer:cell ratio were high which was consistent with the lower cell density.

Thus lack of trace elements restricted cell growth and, thereby, polymer production. In addition the pyruvate content of the product was lower than usual but the product rheology and decarboxylation assay were unaffected.

When a similar culture was supplemented in Comparison Example 3 with 0.2 $gl^{-1}$ yeast extract as a source of trace elements, the cell dry weight and polymer production returned to the more usual levels, even at the higher dilution rate of 0.049 $h^{-1}$. However, the pyruvate content of the product was even lower than in the absence of yeast extract and the product failed the decarboxylation assay. As expected the product had a low zinc content. Thus the lack of trace elements causes a reduction in polymer production and pyruvate content which the yeast extract supplement only partially overcomes.

For Comparison Example 4, zinc was omitted. The $E_{540}$ and cell dry weight decreased, resulting in a lower TPM but not affecting the polymer:cell ratio significantly. Furthermore, the $QO_2$ and product pyruvate content were high. Product rheology was satisfactory and decarboxylation after heat treatment was borderline. Thus Zn seems only essential for providing an adequate cell mass.

Example 6

This example is a larger scale fermentation based on the promising results of Example 5. A shake flask containing 100 ml of a 24 h culture of the same strain in MYGP medium was used to inoculate a 20 litre batch fermenter containing the defined medium A of Table 4 with dissolved dextrose monohydrate at carbon source.

Table 4

| | Medium A | Medium B |
|---|---|---|
| Citric acid $gl^{-1}$ | 1.0 | 1.0 |
| Acetic acid ml | 0.45 | 0.45 |
| $Mg(OH)_2$ $gl^{-1}$ | 0.0775 | 0.0775 |
| $Ca(OH)_2$ $gl^{-1}$ | 0.05 | 0.05 |
| $(NH_4)_2 HPO_4$ $gl^{-1}$ | 3.77 | 3.0 |
| Glucose Syrup (Dexyme 081) $gl^{-1}$ | 45 (40$gl^{-1}$ Meritose for batch 1) | 59 |
| Glucose equivalent $gl^{-1}$ | 33.5 - 36 | 44 |
| $K_2HPO_4$ $gl^{-1}$ | 1.0 | 1.0 |
| $Fe SO_4 7H_2O$ $gl^{-1}$ | 0.0356 | 0.0356 |
| $Mn SO_4 4H_2O$ $gl^{-1}$ | 0.0011 | 0.0011 |
| $Zn SO_4 7H_2O$ $gl^{-1}$ | 0.00142 | 0.00142 |
| $Cu SO_4 5H_2O$ $gl^{-1}$ | 0.00025 | 0.00025 |
| $Co SO_4 7H_2O$ $gl^{-1}$ | 0.00029 | 0.00029 |
| $H_3BO_4$ $gl^{-1}$ | 0.00006 | 0.00006 |
| $Mg SO_4 7H_2O$ $gl^{-1}$ | 0.058 | 0.081 |

After a short lag phase of 10 h, the culture grew with a doubling time of 5 h. At 44 h the $E_{540}$ was 4.1 and 18 litre were used to inoculate 350 litre of defined medium in a 1000 litre fermenter.

The initial 350 litre of medium contained 40 $gl^{-1}$ dextrose and 0.015 $gl^{-1}$ S to support good growth of the culture. Glucose syrup then replaced dextrose as the carbon source and top up to the working volume of 790 litre began at 30 h when the $E_{540}$ and TPM were 3.5 and 11.3 $gl^{-1}$, respectively. Continuous operation with a dilution rate of 0.04 $h^{-1}$ began at 48.5 h when the $E_{540}$ reached 6.4 and the TPM was 25.6 $gl^{-1}$. The residual nitrogen concentration at this time was almost zero. Medium B was then used, in which the sulphur concentration was reduced to 0.012 $gl^{-1}$ to avoid nitrogen limitation.

The TPM rose steadily to 31.9 $gl^{-1}$ during the fermentation and the polymer : cell ratio was 9.65. Furthermore, the pyruvate concentration of the product remained consistently high at around 6%. These much improved data were associated with a low DOT in the culture. To avoid oxygen-limitation, however, the air flow was increased from 1000 litre $min^{-1}$ to 1250 litre $min^{-1}$ at 143 h and over 40 h the DOT increased 3-fold. During this time the $QO_2$ decreased by 25%, the polymer: cell ratio fell to 6.5 and a small decrease in the product pyruvate concentration occurred.

It was possible to maintain a true steady state for 40 h.

Colony variants of the X.campestris were not observed with routine sampling onto MYGP agar plates during the run.


The fermentation conditions and product analyses for towards the end of the run are given in Table 5.

## Table 5

Fermentation Data

| | |
|---|---|
| Product concn $(gl^{-1})$ | 26.9 |
| Cell mass (E 540 units) | 4.8 |
| Cell mass $(gl^{-1})$ | 3.6 |
| Productivity $g(lh)^{-1}$ | 1.13 |
| $qO_2$ m.moles $(g\ cell.h)^{-1}$ | 6.31 |
| Broth rheology $K(cp)$ / $n$ | 37007 / 0.19 |

Product (non heat-treated, lab-precipitated)

| | |
|---|---|
| Nitrogen* | 1.37 |
| Pyruvate* | 6.7 |
| Decarboxylation* | 4.35 |
| Sulphated Ash*(%) | 10.3 |
| Moisture (%) | 9.2 |
| Rheology (1.0% salt $K(cp)$ / $n$ | 13272 / .12 |
| (0.1% salt $K(cp)$ / $n$ | .77 / .52 |

* Corrected for moisture

Broth collected from the fermenter was treated by heat treatment at 125 to 126°C for a residence time of 1.7 to 1.8 min, followed by precipitation in a screw precipitator using isopropyl alcohol. After the precipitation, the xanthan gum was pressed, pelletized, dried and milled.

14.5 kg of milled material was obtained.

This had a consistency index of 14,5000 with $\underline{n}$ = 0.1 and gave an analysis of 11.65% ash, 1.69% nitrogen, 4.29% decarboxylation and 5.3% pyruvate.

Examples 7 and 8

Using the apparatus and procedure of Examples 1 and 5, a shake flask culture (100 ml) was used as an inoculum. Cultures were grown for 24 h before introduction of a continuous flow of medium of the composition indicated in Table 6.

Table 6

Example

| Component $gl^{-1}$ | | |
|---|---|---|
| Citric acid | 1.0 | 1.0 |
| Acetic acid | $0.45\ ml.l^{-1}$ | $0.45\ ml.l^{-1}$ |
| $Ca(OH)_2$ | 0.05 | 0.05 |
| $Mg(OH)_2$ | 0.0775 | - |
| $MgSO_4.7H_2O$ | 0.058 | 0.2 |
| $K_2HPO_4$ | 1.0 | 1.0 |
| $(NH_4)_2HPO_4$ | - | 1.0 |
| Glucose syrup | 52 | 55 |
| Trace elements (1) | $0.05\ ml.l^{-1}$ | $0.05\ ml.l^{-1}$ |
| Trace elements (4) | $0.40\ ml.l^{-1}$ | $0.40\ ml.l^{-1}$ |
| Yeast extract | 4.0 | 1.5 |

Composition of the Trace Elements

Solutions (1) and (4)

Solution (1)

| | | |
|---|---|---|
| $MnSO_4.4H_2O$ | - | 22.0g |
| $ZnSO_4.7H_2O$ | - | 28.40g |
| $CuSO_4.5H_2O$ | - | 5.00g |
| $CoSO_4.7H_2O$ | - | 5.66g |
| $H_3BO_4$ | - | 1.20g |

Solution (4)

| | | |
|---|---|---|
| $FeSO_4.7H_2O$ | - | 89.0g |
| Citric acid | - | 50.0g |

Both solutions were prepared as a stock solution in 1 l distilled water.

The impeller speed was set between 400 and 625 $rev.min.^{-1}$ to obtain a dissolved oxygen tension (DOT) between 10 and 30% of air saturation. The air flow was set between 2.0 and 2.5 litre $min.^{-1}$.

For heat treatment, broth was diluted 1 : 1 with distilled water and heat treated at 125° C for 2 min.

The results are given in Table 7.

Table 7

| | Example | |
|---|---|---|
| | 7 | 8 |
| **Fermentation Data** | | |
| Polymer : cell $\frac{\text{(TPM-Cells)}}{\text{cells}}$ | 1.0 : 1 | 7.2 : 1 |
| TPM $(gl^{-1})$ | 24.5 | 23.0 |
| Productivity $(gl^{-1}h^{-1})$ | 1.25 | 0.92 |
| $qO_2$ (m.moles $(g.cells.h)^{-1}$) | 8.71 | 5.22 |
| Cell dry weight $(gl^{-1})$ | 2.1 | 2.80 |
| $E_{540}$ | 4.00 | 4.00 |
| Broth viscosity, K(n)mPas | 18542(.23) | 33805(.17) |
| Conversion efficiency (%) | 77 | 43 |
| **Product Data** | | |
| Lab ppt { N % | 1.17 | – |
| Consistency index (1.0%) 1% NaCl | 10928(.13) | – |
| K(mPasn)(flow 0.3% | 750(.33) | – |
| behaviour index n)(0.1%) | 52(.59) | – |
| Heat treated ppt { N% | 1.05 | 1.11 |
| Ash % | 9.43 | 10.52 |
| Moisture % | 6.38 | 10.15 |
| Pyruvate % | 5.85 | 3.80 |
| Decarboxylation % | 4.02 | 3.9 |
| Consistency index (1.0%) 1% NaCl | 16765(.10) | 13781(.10) |
| K(mPasn)(flow 0.3% | 1209(.24) | 1199(.26) |
| behaviour index n)(0.1%) | 116(.46) | 103(.49) |

For these examples, yeast extract was used as partial or sole nitrogen source in cultures presumed to be nitrogen-limited. A satisfactory fermentation resulted. The product from Example 7 obtained met nitrogen specification but not decarboxylation specification, probably due to the high concentration of residual glucose in the fermentation. Viscosity after heat treatment was satisfactory as was pyruvate content.

Similar results were obtained in Example 8 when ammonium and yeast extract were used jointly as nitrogen sources in a nitrogen-limited culture. Pyruvate content was lower but within specification,

CLAIMS:

1.   A process for production of xanthan gum by continuous aerobic fermentation of an assimilable carbon source by an organism of the genus xanthomonas in a nutrient medium the improvement which comprises limiting the trace elements Fe, Mn, Zn, Cu, Co, and B as follows:

|    | ppm |  |
|----|--------|--------|
| Fe | 0.1 | - 10 |
| Mn | 0.01 | - 1 |
| Zn | 0.01 | - 1 |
| Cu | 0.001 | - 1 |
| Co | 0.001 | - 1 |
| B | 0.0005 | - 1 |

with the proviso that the ratio Re: Mn ranges from 40: 1 to 2: 1.


2.   The process of claim 1 wherein the trace elements Fe, Mn, Zn, Cu, Co, and B are limited as follows:

|    | ppm |  |
|----|--------|--------|
| Fe | 0.5 | - 5 |
| Mn | 0.1 | - 1 |
| Zn | 0.1 | - 0.5 |
| Cu | 0.01 | - 0.1 |
| Co | 0.01 | - 0.1 |
| B | 0.005 | - 0.05 |

3.   The process of claim 1 wherein the nutrient medium is prepared from demineralized water, the carbon source is a carbohydrate, and the medium comprises nitrogen from a complex, naturally-occurring mixture.

4.    The process of claim 1 wherein the medium further comprises water-soluble inorganic salts of phosphorus, magnesium, calcium, potassium, and sulphur.

5.    The process of claim 1 wherein the dilution rate is 0.01 - 0.12/hr.

6.    The process of claim 5 wherein the dilution rate is 0.02 - 0.08/hr and the fermentation temperature is 25-35$^o$C.

7.    The process of claim 6 wherein the dilution rate is 0.04 - 0.6/hr and the fermentation temperature is 25-32$^o$C.

8.    The process of claim 1 comprising the further step of heating the fermented medium to 100-30$^o$C for 1-15 minutes prior to recovery of the xanthan gum.

9.    The process of claim 8 wherein the fermented medium is heated to 120-125$^o$C for 2 minutes.

10.    The process of claim 1, wherein the organism is X.campestris.

11.    The process of claim 10, wherein the organism has the identifying characteristics of X.campestris ATCC 13951.

0066377

**European Patent Office**

# EUROPEAN SEARCH REPORT

Application number

EP 82 30 2331

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | GB-A-2 008 138  (TATE & DYLE)<br>* Claims 1-4; example 1 *<br><br>--- | 1 | C 12 P   19/06 |
| P,X | EP-A-0 045 569  (STANDARD OIL)<br>* Claims 1-13; example 1 *<br><br>----- | 1-5,10 | |
| | | | **TECHNICAL FIELDS SEARCHED (Int. Cl. 3)**<br><br>C 12 P   19/00 |

The present search report has been drawn up for all claims

| Place of search | Date of completion of the search | Examiner |
|---|---|---|
| THE HAGUE | 02-09-1982 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82